# EUROPEAN PATENT APPLICATION

(11) **EP 2 548 638 A2**
(43) Date of publication of application: **23.01.2013**
(21) Application number: 12175560.7
(22) Date of filing: 09.07.2012
(51) Int. Cl.: B01J 13/04, A61K 9/51

(54) **A method for producing polymer-encapsulated nanoparticles**

(30) Priority: 20.07.2011 MY PI2011003395
(71) Applicant: Universiti Putra Malaysia, 43400 Serdang, Selangor (MY)
(72) Inventor: Yunus, Robiah, 43400 Serdang Selangor (MY); Kalani, Mahshid, 43400 Serdang Selangor (MY)
(74) Representative: O'Connell, Maura

(57) **Abstract**

The present invention relates to a method for producing polymer-encapsulated nanoparticles comprising the steps of: pumping supercritical fluid into a pressurised vessel, at a predetermined pressure; spraying a solution comprising a compound to be encapsulated, a polymer and an organic solvent into the vessel; collecting precipitated particles on a filter; and washing the particles with the supercritical fluid to remove residual solvent; characterised in that: the difference between the melting point of the polymer and the melting point of the compound to be encapsulated is not more than 10 °C.

## Description

### Field of the Invention

This invention relates to a method for producing polymer-encapsulated particles, and more particularly a method for producing polymer-encapsulated nanoparticles using supercritical antisolvent.

### Description of Related Arts

Polymer-encapsulated particles are particles of a compound coated with a layer of a polymer. These particles can have various applications, depending on the compound that has been encapsulated and the choice of polymer for encapsulating the compound. The polymer-encapsulated particles may be used in various industries such as the agricultural or chemical industries. In particular, these polymer-encapsulated particles are of great interest in the pharmaceutical industry. The compound to be encapsulated is typically a drug compound, while the polymer is a biodegradable and biocompatible polymer. By encapsulating the drug compound with the polymer, it is possible control the delivery and release of the drug. For example, drug encapsulation can enable the slow release of soluble drugs in water, the rapid release of low-solubility drugs, drug delivery to specific sites, and the delivery of more than one agent with the same formulation and system based on soluble or degradable carriers that are easily eliminated. Controlled drug delivery using polymer-encapsulated drug particles can therefore improve drug bioavailability, increase effectiveness of the drug and reduce risk of adverse side effects. However, it is also necessary to control the particle size and its distribution for efficient drug delivery. Smaller particles are preferred as they increase the total surface area, leading to better bioavailability. Small particles with narrower particle size distribution provide better flexibility of administration.

The supercritical antisolvent (SAS) method is a common method for encapsulating compounds to produce polymer-encapsulated particles. In the prior art, various studies have been done to optimise the supercritical antisolvent process to improve the characteristics of the resulting polymer-encapsulated particles.

In the study by Taki and Charbit, 2001, there was disclosed a supercritical antisolvent (SAS) method for preparation of a herbicide (diuron) loaded in amorphous microparticles of a biodegradable polymer (L-polylactic acid, LPLA). The method provided therein produced needle-like crystals with a mean length of 500 µm. This cited art also disclosed that the concentrations of the compound to be encapsulated (diuron) and the polymer (LPLA) had a major effect on the morphology and shape of the resulting encapsulated particles.

Another prior art (Patomchaiviwat, Paeratakul and Kulvanich, 2008), provided a method for producing inhalable microparticles containing rifampicin and L-polylactic acid (LPLA). In this study, the effect of polymer content and operating conditions, temperature, pressure, carbon dioxide molar fraction, and concentration of solution on product characteristics was investigated. The results had average particle sizes of 3.27 - 5.25 µm.

In yet another prior art by Lee *et al.,* 2006, a method was disclosed to encapsulate bupivacaine hydrochloride using poly(D,L-lactic-co-glycolic acid) (PLGA) and poly(L-lactic acid) (PLLA). Using the supercritical antisolvent method, microspheres were prepared using polymer mixtures comprising different ratios of PLGA to PLLA. Two different levels of bupivacaine hydrochloride were studied (5 and 10%). The results produced were microspheres having geometric mean diameter of 4 - 10 µm.

It can therefore be seen that the methods used in the prior arts are directed towards optimising the parameters related to equipment, material crystallinity or composition. However, achieving the optimum result with manipulation of the mentioned parameters is very difficult or expensive. Therefore, there exists a need to provide an alternative method to produce polymer-encapsulated particles having fine and uniform morphology with minimised particle size.

### References

- Taki, E. B. S., and Charbit, G. 2001. Controlled release system formed by supercritical anti-solvent coprecipitation of a herbicide and a biodegradable polymer. J. of Supercritical Fluids 21:61-70.
- Patomchaiviwat, V., Paeratakul, O., and Kulvanich, P. 2008. Formation of inhalable rifampicin-poly(L-lactide) microparticles by supercritical anti-solvent process. AAPS PharmSciTech 9:1119-29.
- Lee, S., Kim, M. S., Kim, J. S., Park, H. J., Woo, J. S. 2006. Controlled delivery of a hydrophilic drug from a biodegradable microsphere system by supercritical anti-solvent precipitation technique. J. of Microencapsulation 23:741-9.

### Summary of Invention

It is an objective of the present invention to provide a method for producing polymer-encapsulated nanoparticles.

It is also an objective of the present invention to provide a method for minimising the particle size of polymer-encapsulated nanoparticles.

It is another objective of the present invention to provide a method to produce polymer-encapsulated nanoparticles having narrow particle size distribution.

It is yet another objective of the present invention to provide a method to produce polymer-encapsulated nanoparticles having low agglomeration.

It is yet another objective of the present invention to provide a method to produce polymer-encapsulated nanoparticles having spherical shape.

Accordingly, these objectives may be achieved by following the teachings of the present invention. The present invention relates to a method for producing polymer-encapsulated nanoparticles comprising the steps of: pumping supercritical fluid into a pressurised vessel, at a predetermined pressure; spraying a solution comprising a compound to be encapsulated, a polymer and an organic solvent into the vessel; collecting precipitated particles on a filter; and washing the particles with the supercritical fluid to remove residual solvent; characterised in that: the difference between the melting point of the polymer and the melting point of the compound to be encapsulated is not more than 10 °C.

### Brief Description of the Drawings

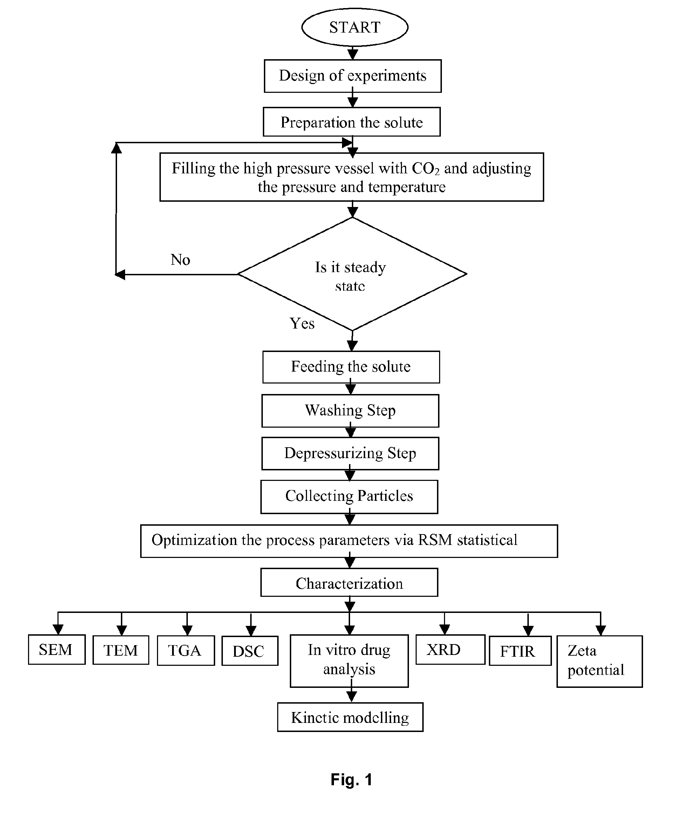

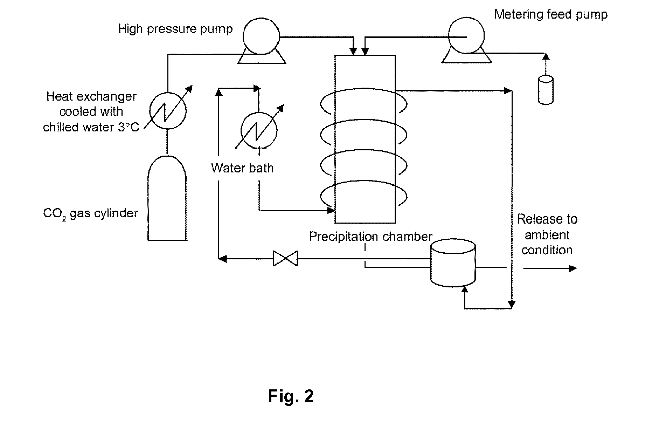

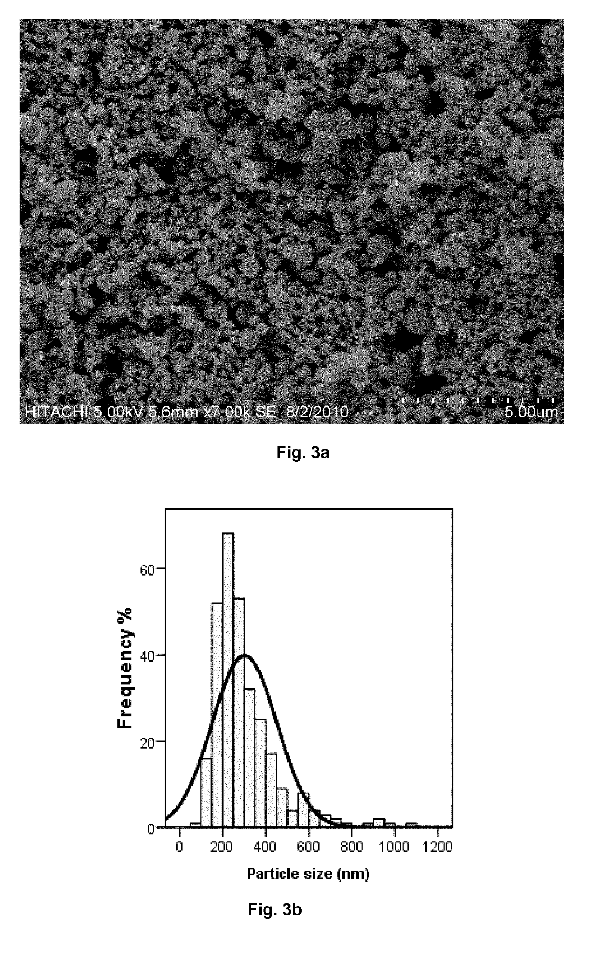

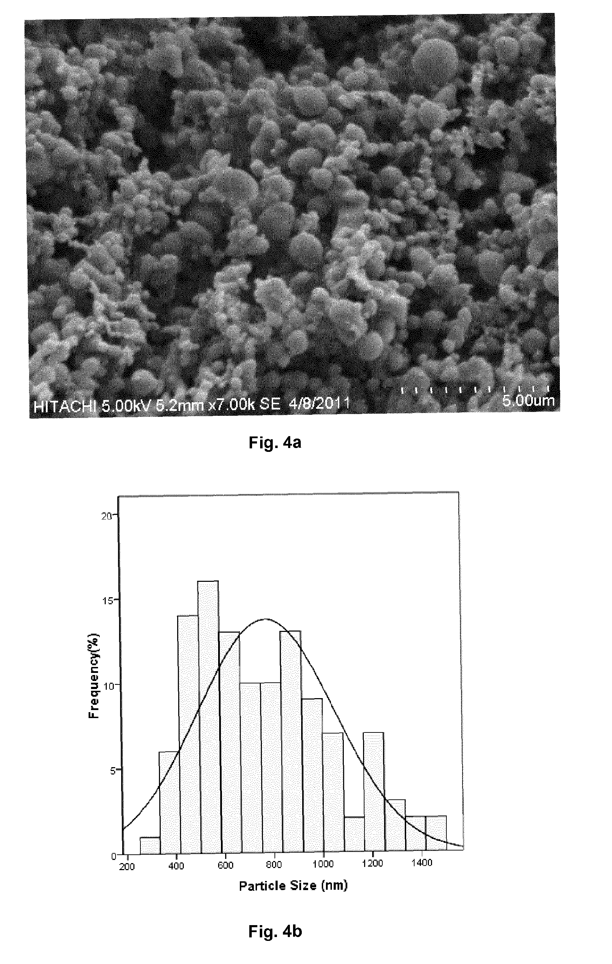

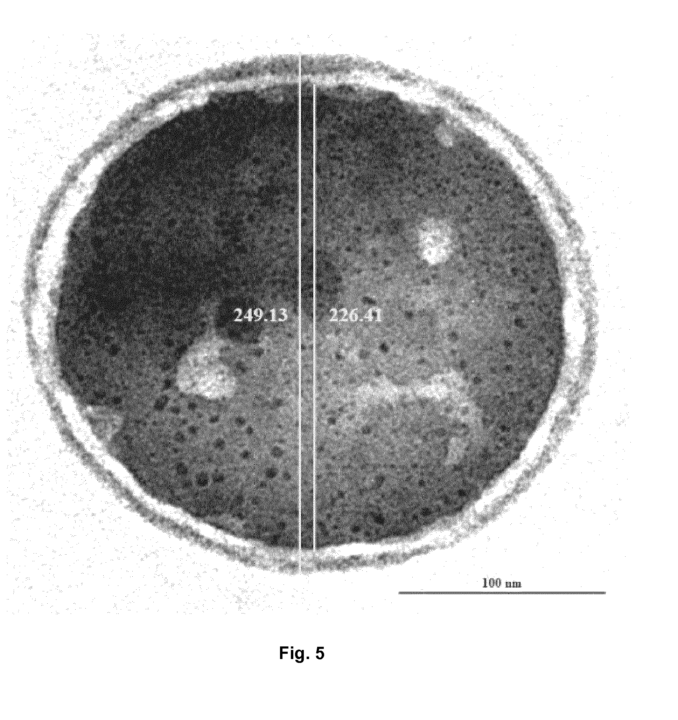

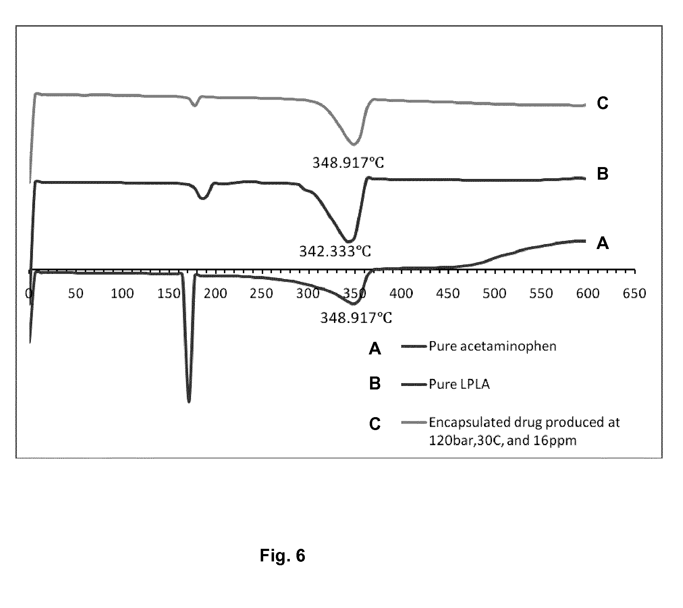

The features of the invention will be more readily understood and appreciated from the following detailed description when read in conjunction with the accompanying drawings of the preferred embodiment of the present invention, in which:
● Fig. 1 is a flowchart diagram of an example of the method of the present invention;
● Fig. 2 is a schematic diagram of the apparatus for the supercritical antisolvent process;
● Fig. 3a is an SEM image of LPLA-encapsulated paracetamol nanoparticles;
● Fig. 3b is a graph showing the particle size distribution of LPLA-encapsulated paracetamol nanoparticles;
● Fig. 4a is an SEM image of LPLA-encapsulated diclofenac sodium nanoparticles;
● Fig. 4b is a graph showing the particle size distribution of LPLA-encapsulated diclofenac sodium nanoparticles;
● Fig. 5 is a TEM image showing the internal structure of an LPLA-encapsulated paracetamol nanoparticle;
● Fig. 6 is a graph showing a comparison of the melting points and decomposition points of pure paracetamol (A), pure LPLA (B), and LPLA-encapsulated paracetamol nanoparticles (C).

### Detailed Description of the Invention

As required, detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting but merely as a basis for claims. It should be understood that the drawings and detailed description thereto are not intended to limit the invention to the particular form disclosed, but on the contrary, the invention is to cover all modification, equivalents and alternatives falling within the spirit and scope of the present invention as defined by the appended claims. As used throughout this application, the word "may" is used in a permissive sense (i.e., meaning having the potential to), rather than the mandatory sense (i.e., meaning must). Similarly, the words "include," "including," and "includes" mean including, but not limited to. Further, the words "a" or "an" mean "at least one" and the word "plurality" means one or more, unless otherwise mentioned. Where the abbreviations or technical terms are used, these indicate the commonly accepted meanings as known in the technical field. As used herein, the term "organic solvent" means a solvent including an organic compound or including a mixture of organic compounds. For ease of reference, common reference numerals will be used throughout the figures when referring to the same or similar features common to the figures. The present invention will now be described with reference to Figs. 1-6.

The present invention relates to a method for producing polymer-encapsulated nanoparticles. More particularly, the present invention provides a method for minimising the particle size of polymer-encapsulated nanoparticles.

The method for producing polymer-encapsulated nanoparticles comprises the steps of:
pumping supercritical fluid into a pressurised vessel, at a predetermined pressure;
spraying a solution comprising a compound to be encapsulated, a polymer and an organic solvent into the vessel;
collecting precipitated particles on a filter; and
washing the particles with the supercritical fluid to remove residual solvent;
characterised in that:
   the difference between the melting point of the polymer and the melting point of the compound to be encapsulated is not more than 10 °C.

In a preferred embodiment of the present invention, the supercritical fluid is carbon dioxide.

In a preferred embodiment of the present invention, the supercritical fluid is pumped at a flow rate of 15 mL/min.

In a preferred embodiment of the present invention, the predetermined pressure is 120 bar.

In a preferred embodiment of the present invention, the polymer is a semicrystalline biodegradable polymer.

In a preferred embodiment of the present invention, the concentration of the polymer in the solution is 16 mg/mL.

In a preferred embodiment of the present invention, the concentration of the compound to be encapsulated is 0.5 mg/mL.

In a preferred embodiment of the present invention, the organic solvent comprises a solvent for the polymer and a solvent for the compound to be encapsulated.

In a preferred embodiment of the present invention, the solvent for the polymer is methylene chloride.

In a preferred embodiment of the present invention, the solvent for the compound to be encapsulated is acetone.

In a preferred embodiment of the present invention, the organic solvent comprises methylene chloride and acetone in a ratio of 3:2 by volume.

In a preferred embodiment of the present invention, the solution is sprayed into the vessel at a flow rate of 1.75 mL/min.

In a preferred embodiment of the present invention, the solution is sprayed into the vessel at a feed volume of 20 mL.

In a preferred embodiment of the present invention, the method is carried out a temperature of 30 °C to 60 °C.

Below is an example of the method for producing polymer-encapsulated nanoparticles from which the advantages of the present invention may be more readily understood. It is to be understood that the following example is for illustrative purpose only and should not be construed to limit the present invention in any way.

### Example

In the following example of the present invention, a study is conducted on a compound to be encapsulated. The first compound to be encapsulated is paracetamol (99.9%), with a melting point of 169-172 °C. The second compound to be encapsulated is diclofenac sodium, with a melting point of 284 °C. The polymer used in this example is poly L-lactic acid (LPLA) with a melting point of 173-178 °C (intensive viscosity 2.0 dL/g). High-pressure liquid chromatography grade methylene chloride (dichloromethane, DCM) and high-pressure liquid chromatography grade acetone are used as the organic solvent. Purified grade carbon dioxide is used as the supercritical fluid.

### Preparation of polymer-encapsulated nanoparticles

Referring to Fig. 1, a chiller (Thermo Scientific, USA) and a heat exchanger were used to liquefy carbon dioxide from a gas cylinder. The pressure and temperature of liquid carbon dioxide increased to the desired supercritical carbon dioxide density in the reactor (490 mL) using a high pressure pump (Thar SCF P-50A, USA) and a water bath. The chiller temperature was decreased to 3 °C for protection of the high pressure pump.

Supercritical fluid was pumped into a pressurised vessel and the system was equilibrated for 20 minutes via a regulator valve. The solution comprising the compound to be encapsulated, the polymer and the organic solvent was delivered at a constant flow rate equal to 1.75 mL/min using a metering pump (Chrom Tec Series III, USA) and the flowrate is controlled via a flowmeter. A constant feed delivery volume of 20 mL was injected to the reactor via a capillary nozzle with a diameter of 0.2 micron installed on top of the reactor when the supercritical fluid density was at equilibrium condition. The solvent diffused rapidly from the solution droplets into the bulk supercritical fluid, precipitating the solute that comprised the compound to be encapsulated in the biodegradable polymer. To ensure that the residual solvent was fully removed, the flow rate of supercritical fluid continues for 1.5 hours after feed delivery. Then the precipitation vessel was slowly depressurized to the ambient condition and the particles were collected from inside the reactor.

Fig. 2 shows a schematic diagram of the apparatus for the supercritical antisolvent process of the present invention. The process parameters for this example are shown in Table 1 below.

**Table 1: Process parameters**

| **Process parameters** | **Value range** | **Preferred value** |
|---|---|---|
| Pressure (bar) | 80-120 | 120 |
| Temperature (°C) | 30-60 | 30 |
| Polymer concentration (mg/mL) | 12-32 | 16 |
| Carbon dioxide flow rate (mL/min) | 15 | 15 |
| Feed flow rate (mL/min) | 1.75 | 1.75 |
| Feed delivery volume (mL) | 20 | 20 |
| Ratio of DCM/acetone | 3:2 | 3:2 |
| Compound to be encapsulated concentration (mg/mL) | 0.5 | 0.5 |

### Results

The polymer-encapsulated nanoparticles of paracetamol and diclofenac sodium produced using the method above were compared. Referring to Figs. 2a and 2b, it can be seen that the encapsulated paracetamol nanoparticles are very fine and spherical in shape, with a mean particle size of 300 nm. The resulting nanoparticles were coated with a nanolayer of polymer of about 20 nm thickness. The particle size distribution was narrow and no agglomeration was observed.

In comparison, the encapsulated diclofenac sodium nanoparticles shown in Figs. 3a and 3b have reduced morphology and are less spherical than the paracetamol nanoparticles. The mean particle size achieved for the encapsulated diclofenac sodium nanoparticles in LPLA was 770 nm, which is more than twice the mean particle size of encapsulated paracetamol. A wider, less uniform distribution of particle size was observed in the encapsulated diclofenac sodium nanoparticles, when compared with the encapsulated paracetamol nanoparticles.

The results indicate that when the thermal characteristics of the compound to be encapsulated and polymer are closely matched, the size of the resulting polymer-encapsulated nanoparticles can be minimised. In the present invention, the difference between the melting point of the polymer and the melting point of the compound to be encapsulated is not more than 10 °C. When the difference between the melting point of the polymer and the melting point of the compound to be encapsulated was increased, it was found that the particle size increased and particle morphology reduced. In the present example, paracetamol and LPLA have similar decomposition points as well, but the effect of the matching in the decomposition points of the polymer and the compound to be encapsulated may not be as significant because the polymer and the compound do not decompose during the process of the present invention.

The internal structure of the encapsulated compound to be encapsulated nanoparticle produced using the method of the present invention is shown in Fig. 5. Fig. 5 shows a solid phase formed by the polymer coating a core of material formed by the compound to be encapsulated. Fig. 6 shows a comparison of the melting points and decomposition points of pure paracetamol (A), pure LPLA (B), and LPLA-encapsulated paracetamol nanoparticles (C).

Although the present invention has been described with reference to specific embodiments, also shown in the appended figures, it will be apparent for those skilled in the art that many variations and modifications can be done within the scope of the invention as described in the specification and defined in the following claims.

## Claims

1. A method for producing polymer-encapsulated nanoparticles comprising the steps of:
pumping supercritical fluid into a pressurised vessel, at a predetermined pressure;
spraying a solution comprising a compound to be encapsulated, a polymer and an organic solvent into the vessel;
collecting precipitated particles on a filter; and
washing the particles with the supercritical fluid to remove residual solvent;
**characterised in that**:
the difference between the melting point of the polymer and the melting point of the compound to be encapsulated is not more than 10 °C.

2. A method for producing polymer-encapsulated nanoparticles according to claim 1, wherein the supercritical fluid is carbon dioxide.

3. A method for producing polymer-encapsulated nanoparticles according to claim 1, wherein the supercritical fluid is pumped at a flow rate of 15 mL/min.

4. A method for producing polymer-encapsulated nanoparticles according to claim 1, wherein the predetermined pressure is 120 bar.

5. A method for producing polymer-encapsulated nanoparticles according to claim 1, wherein the polymer is a semicrystalline biodegradable polymer.

6. A method for producing polymer-encapsulated nanoparticles according to claim 1, wherein the concentration of the polymer in the solution is 16 mg/mL.

7. A method for producing polymer-encapsulated nanoparticles according to claim 1, wherein the concentration of the compound to be encapsulated is 0.5 mg/mL.

8. A method for producing polymer-encapsulated nanoparticles according to claim 1, wherein the organic solvent comprises a solvent for the polymer and a solvent for the compound to be encapsulated.

9. A method for producing polymer-encapsulated nanoparticles according to claim 8, wherein the solvent for the polymer is methylene chloride.

10. A method for producing polymer-encapsulated nanoparticles according to claim 8, wherein the solvent for the compound to be encapsulated is acetone.

11. A method for producing polymer-encapsulated nanoparticles according to claim 1, wherein the organic solvent comprises methylene chloride and acetone in a ratio of 3:2 by volume.

12. A method for producing polymer-encapsulated nanoparticles according to claim 1, wherein the solution is sprayed into the vessel at a flow rate of 1.75 mL/min.

13. A method for producing polymer-encapsulated nanoparticles according to claim 1, wherein the solution is sprayed into the vessel at a feed volume of 20 mL.

14. A method for producing polymer-encapsulated nanoparticles according to claim 1, wherein the method is carried out a temperature of 30 °C to 60 °C.
